Europäisches Patentamt

European Patent Office

Office européen des brevets

(11) Publication number: **0 241 000**
**A2**

## EUROPEAN PATENT APPLICATION

(21) Application number: 87105151.2

(22) Date of filing: 07.04.87

(51) Int. Cl.4: **G01N 33/577** ,
//G01N33/49,G01N33/536,G01-
N21/47

(30) Priority: 09.04.86 JP 81873/86

(43) Date of publication of application:
14.10.87 Bulletin 87/42

(84) Designated Contracting States:
AT BE CH DE ES FR GB GR IT LI LU NL SE

(71) Applicant: **WAKO PURE CHEMICAL**
**INDUSTRIES, LTD.**
**10, Doshomachi-3-chome**
**Higashi-ku Osaka(JP)**

(72) Inventor: **Sakata, Yoshitsugu**
**36-7 Hiyoshidai-2-chome**
**Otsu-shi(JP)**
Inventor: **Tsubaki, Kazuyuki**
**19-69, Aza Shireta**
**Yamada Itami-shi(JP)**
Inventor: **Kobatake, Shinzo**
**18-A-10-206, Yamadanishi-2-chome**
**Suita-shi(JP)**
Inventor: **Ushio, Yoshihiro**
**11-3-307, Kitanatsugicho**
**Nishinomiya-shi(JP)**

(74) Representative: **Baillie, Iain Cameron et al**
**c/o Ladas & Parry Isartorplatz 5**
**D-8000 München 2(DE)**

(54) **Method for determination of complements.**

(57) In an optical method for the determination of complements in a living body, the use of a living body sample without dilution and the use of two or more kinds of monoclonal antibodies to human complements as antibodies to the complement to be assayed can make the determination more precise and rapid.

EP 0 241 000 A2

## METHOD FOR DETERMINATION OF COMPLEMENTS

BACKGROUND OF THE INVENTION

This invention relates to an optical method for the determination of human complements using monoclonal antibodies to human complements.

The complement is composed of 20 or more proteins contained in fresh serum and participates in important reactions in living bodies such as protection against infection, immunoreactions, and inflammation. Of the complements, C3 and C4 are contained in serum in especially high concentrations, and the assay of the C3 and C4 contents of serum is an important means for the diagnosis, prophylaxis and prognosis of various diseases.

In recent years, remarkable progress has been made in the method of assay for the complement in body fluids, and there have been developed various assay methods such as SRID, laser nephelometry, turbidimetry, RIA, EIA, etc. Especially, a method of rapid assay for complement by means of a laser nephelometer or an autoanalyzer developed for the biochemical assay of multiple items has been lately developed and becoming now in wide use.

However, the above methods had the following defect. The antibody used in the above methods is generally a polyclonal one and hence the degree of agglutination caused by the antigen-antibody reaction is too high, resulting in too high a turbidity. Therefore, the sample serum should be diluted before being assayed, and such dilution makes the assay procedure complicated and tends to introduce an error into the results of assay.

Furthermore, since the assay by means of an automatic analyzer is performed, as a rule, on an undiluted sample, it was practically impossible to assay the complement content simultaneously with biochemical examination of sugars, lipids, enzymes, and the like on the same sample.

Of the complements, C3 is contained in serum in a particularly high concentration, the ordinary content being 50 to 200 mg/dl. Accordingly, in an optical method of assay such as turbidimetry or nephelometry using polyclonal antibodies derived from an immunized animal, if the assay is carried out on undiluted serum, a large amount of the antibodies are needed, resulting in an extremely high turbidity. Therefore, it was believed utterly impossible to carry out the measurement on undiluted serum. Thus, for conducting the optical measurement, it was necessary to carry out the antigen-antibody reaction in a serum sample of 5 to 30 fold dilution or, in some cases, about l00 fold dilution (e.g., nephelometry).

On the other hand, examples of the methods of clinical chemical analysis using monoclonal antibodies in the antigen-antibody reaction include radioimmunoassay (RIA), enzyme immunoassay (EIA), fluoroimmunoassay (FIA), etc. In these methods, the antigen or antibody being assayed is labeled with an isotope, enzyme or fluorescent substance and after completion of the antigen-antibody reaction the amount of antigen or antibody is indirectly determined through the measurement of the substance used in the labeling, the monoclonal antibody being used in order to improve the precision of assay.

However, in the case of direct optical measurement of the degree of agglutination resulting from the antigen-antibody reaction, no example of the effective use of monoclonal antibodies is found except the case of immunoglobulins described hereinafter.

A reason for this is as follows. In general, in the case of monoclonal antibodies, when the antigen is, for example, C3 which is one of the complements in this invention, C3 has only one antigenic determinant per molecule, so that the C3 molecule can combine with a monoclonal antibody molecule only at one site, and hence the resulting complex will not sufficiently grow in particle size to increase the turbidity, contrary to the case of polyclonal antibodies originated from an animal, wherein many antibody molecules, not predeterminable in number, combine with one C3 molecule, so that polymerization proceeds to increase the particle size of antigen-antibody complex, resulting in an increase in turbidity. Therefore, it has been generally believed that when monoclonal antibodies are used, there is caused no polymerization, and hence no production of turbidity.

Under these circumstances, the present inventors have found that in the case of immunoglobulines, the degree of agglutination caused by the antigen-antibody reaction when monoclonal antibodies are used each alone or in combination of two or more of them can be measured directly and optically, and have previously applied for a patent (EP-A-0l6l638). This finding was obtained on the basis of the notion that since in the case of immunoglobulins, the antigen molecule has two binding sites for antibody molecules, measurable turbidity, though slight, is probably produced even when a monochlonal antibody is used alone. In fact, in the case of immunoglobulins, measurable turbidity was observed even when a monoclonal antibody was used alone. On the other hand, the complement of this invention has only one antigenic determinant per molecule, so that the complement molecule can combine with a monoclonal antibody molecule only at one site. Therefore, no turbidity is produced when a monoclonal antibody is used alone, and it did not seem probable at all that measurable turbidity is produced when two or more monoclonal antibodies are used in combination.

SUMMARY OF THE INVENTION

In order to solve the above problems in the optical methods for the determination of complements, the present inventors have made an extensive study and have consequently found that employment of combinations of two or more kinds of monoclonal antibodies can give a turbidity suitable and sufficient for the optical measurement even in the case of an undiluted sample of body fluids such as serum, and permits easy and efficient assay of complements by nephelometry or turbidimetry, whereby this invention has been accomplished.

Furthermore, in the course of study on the relationship between monoclonal antibodies to complements and turbidity, the present inventors have found that although no turbidity is observed when a monoclonal antibody is used alone, turbidity is unexpectedly observed when a combination of two or more kinds of monoclonal antibodies is used, and that the degree of turbidity can be adjusted by use of a proper combination of monoclonal antibodies, so that a very suitable sensitivity can be obtained, whereby this invention has been accomplished.

An object of this invention is to provide a method which permits easy and efficient quantitation of complements by an optical assay method such as nephelometry, turbidimetry, or the like by use of an undiluted sample of body fluids such as serum.

This invention provides a method for the determination of complements in a living body sample, comprising exposing an antigen-antibody complex produced by the antigen-antibody reaction to light, and measuring the change of optical properties, which is characterized by using the living body sample without dilution, and using two or more kinds of monoclonal antibodies to human complements as antibodies to the complement being assayed.

BRIEF DESCRIPTION OF THE DRAWINGS

Fig. I is a calibration curve obtained in Example 2 representing a relationship between the quantity of C4 and the absorbance (OD).

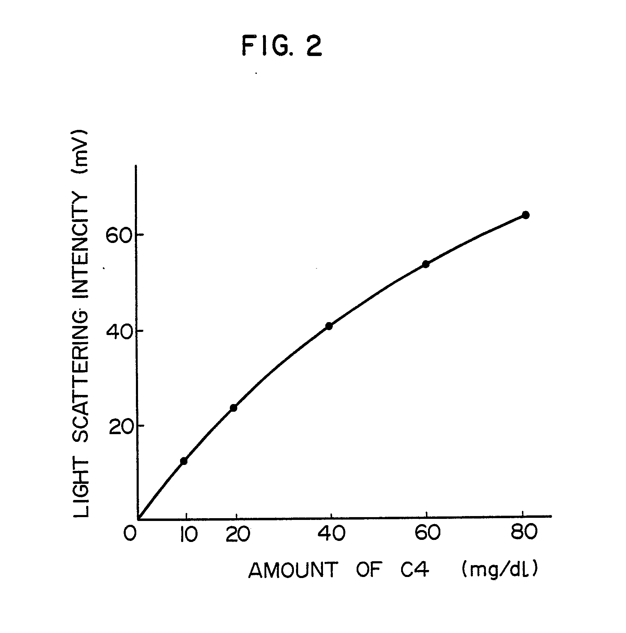

Fig. 2 shows a calibration curve obtained in Example 3 representing a relationship between the quantity of C4 and light scattering intensities (mV).

DESCRIPTION OF THE PREFERRED EMBODIMENTS

When monoclonal antibodies are used for optical determination of complements, even if two or more monoclonal antibodies are used, the degree of turbidity is considerably different compared with the case of using polyclonal antibodies. But in the present invention, since two or more kinds of monoclonal antibodies are used, C3 or C4 in a sample can provide the degree of turbidity sufficient for measuring when a living body sample such as serum is used without dilution.

According to the method of this invention, the determination can be carried out on an undiluted sample of body fluids such as serum, and consequently, complicated procedure and the error of measurement associated with dilution can be eliminated, resulting in an improvement of the precision. Moreover, it becomes very easy to include the complement assay into multi-item biochemical examination for sugars, lipids, enzymes, and so on, carried out on a single sample by means of an automatic analyzer, so that the field of application of the present method is greatly extended.

The first stage in achieving the object of this invention is the formation of monoclonal hybridoma excreting the antibodies, which comprises essentially the following three steps:

1. Immunization
2. Cell fusion
3. Selection and cloning of the hybridoma

The procedures in each step are described below in the order indicated.

The antigen used in the immunization is purified C3 or C4 separated from human serum.

C3 or C4 is dissolved in physiological saline, buffer, or the like and injected to mourse or rat preferably at a dose of I to 300 μg. The immunization is performed several times at predetermined intervals. The primary immunization is generally performed with the aid of an adjuvant. As the adjuvant, Freund's adjuvant, alum, etc. are used. The administration is repeated at intervals of 2 to 4 weeks. The final immunization is performed by administering intraperitoneally or intravenously a solution of C3 or C4 in physiological saline without using an adjuvant. As the animal being immunized, rat or mouse is generally used. This is because the selection of strain is dependent upon the established tumor cell strain used in the cell fusion. Of the mouse strains, there is frequently used BALB/c which is related to an established tumor cell strain producing no imunoglobulin. Two to four days after the final immunization, the lymph node or the spleen is excised from the animal body and the lymphocyte obtained therefrom is used in the cell fusion.

On the other hand, the tumor cell strain used in the cell fusion is P3-X63-8AZ-Ul, P3-NSl-l or the like producing no immunoglobulin. In the cell fusion, the lymphocyte is used in an amount of 5 to 20 times that of the tumor cells. After washing with MEM medium, McCoy medium, RPMI l640 medium, an isotonic buffer, or the like, the lymphocyte and the tumor cell are pelleted by centrifugation. The pellets are loosened and then subjected to cell fusion in the presence of Sendai virus (HVJ) or polyethylene glycol (PEG). There is usually used 0.5 to 2 ml of a 40 to 60% solution of PEG having an average molecular weight of I,000 to 8,000 which is easy to handle. A small amount of dimethyl sulfoxide or the like is, in some cases, added together with PEG to accelerate the fusion but it is not essential. After the addition of a PEG solution, the cell fusion is carried out for about I to l0 minutes, and then l0 to 50 ml of MEM medium, RPMI l640 medium or the like is slowly added to terminate the reaction. Immediately after termination of the cell fusion, the reaction mixture is centrifuged to remove the supernatant. The fused cells are suspended in MEM or RPMI l640 medium containing 5 to 20% of fetal calf serum (FCS), and the resulting suspension is fed to a 24-wells culture plate in an amount of I ml per well so that $I \times l0^5$ to $5 \times l0^6$ lymphocytes may be present in each well. Whichever medium is used, it is desirable to add feeder cells. As the feeder cells, there are used, for example, thymus cells or spleen cells of rat or mouse of the same line. The feeder cells are added so as to adjust the concentration thereof in the medium to $(0.5-2) \times l0^6$/ml. The culture medium is replaced stepwise by RPMI l640 medium (or MEM medium) containing $I \times l0^{-4}$ M hypoxanthine, $4 \times l0^{-7}$ M aminopterin, and $1.6 \times l0^{-5}$ M thymidine, that is HAT medium. The replacement by HAT medium is generally carried out in the following manner. On the day succeeding the day of feeding the suspension to each well of the culture plate, HAT medium is added to each well in the same amount as that of the suspension fed to each well. On the next day, one-half of the medium in each well is replaced by an equal volume of HAT medium. Then, every 2 or 3 days, one-half of the medium is replaced by an equal volume of HAT medium. On the l0-l4th day after the fusion, one-half of the medium is replaced by an equal volume of HT medium (HAT medium minus aminopterin). Then, every I-3 days, one-half of the medium is replaced by an equal volume of ordinary medium containing no HAT. The supernatant of the culture medium in the wells where the growth of fused cells (hybridoma) is actively in progress is examined by various analyzing techniques, for example, RIA and ELISA to select the hybridoma producing the intended antibody. The selected hybridoma is then cloned. Methods for the cloning include a method using a fluorescent activated cell sorter (FACS), a method of picking up a colony from soft agar, a method of limiting dilution which is widely used, etc. Whichever method is employed, the cloning is repeated twice or more to secure a perfect monoclone.

Once monoclones have been established, various monoclonal antibodies to respective human complements can be obtained by culturing the cells of each clone in vitro or in vivo. Although the culture may be carried out either in vitro or in vivo, the culture in vivo is preferred because of a much higher antibody titer.

Methods for the determination of complements using the monoclonal antibodies include turbidimetry, nephelometry, etc. When the assay is carried out by turbidimetry, a known automatic analyzer or a spectrophotometer can be used, and the wave length is preferably in the range of 340 to 800 nm, though not critical. When the assay is carried out by nephelometry, there can be used, for example, a nephelometer with laser as a light source which is now widespread, but the invention is not limited to any particular light source or the method of detection.

As the buffers used in the assay, there can be used all of those which are generally used, such as Tris buffer, phosphate buffer, and veronal buffer. The pH is prepferably in the range of 6.0 to 9.0, though not critical.

The type and number of monoclonal antibodies used in the assay are not critical, and it is sufficient that a proper combination of two or more kinds of monoclonal antibodies is used depending upon the required sensitivity.

4

Although the using amount of the monoclonal antibodies is not critical, they are used usually in the concentration range of 0.0l to l mg per ml of the antibody solution.

Complements which can be assayed by the method of this invention include, for example, human C3 and C4 which, among the complements in serum, are contained in serum in high concentrations.

This invention is illustrated below in further detail with reference to Referential Examples and Examples, which are not by way of limitation but by way of illustration.

EXAMPLES

Referential Example l

Preparation of monoclonal anti-C3 antibody and hybridoma producing the same
l. Immunization.

An immunogen was prepared by mixing a solution of l00 $\mu$g of human C3 in 0.l ml of a 0.l5 M sodium chloride solution with 0.l ml of complete Freund's adjuvant. To a BALB/c mouse (female, 6 weeks of age) was intraperitoneally administered 0.2 ml of the emulsion thus obtained. After 4 weeks, a solution of l00 $\mu$g of human C3 in 0.2 ml of a 0.l5 M sodium chloride solution was injected into the tail vein of the mouse.
2. Cell fusion

Three days after the final immunization, the spleen was removed from the immunized mouse and minced in a plastic Petri dish containing l0 ml of RPMI l640 medium to obtain splenocytes. The splenocytes were washed three times with RPMI l640 medium by repeated centrifugation (l,000 rpm, l0 minutes). Then, l $\times$ l0$^8$ splenocytes and l $\times$ l0$^7$ mouse myeloma cells P3-NSl-l were mixed in a test tube and then precipitated by centrifugation. After the supernatant was removed by suction, the precipitate was lightly loosened and l ml of 50% polyethylene glycol (average molecular weight: 6000) was added to the loosened precipitate. Cell fusion was carried out at room temperature for l minute, while rotating the test tube. Every 30 seconds, l ml of RPMI l640 medium was added over a period of 5 minutes to stop the fusion reaction. The reaction mixture was immediately centrifuged (l,000 rpm, 5 minutes), after which the supernatant was removed and the fused cells were suspended in 50 ml of RPMI l640 medium containing l $\times$ l0$^6$/ml of feeder cells and 20% of fetal calf serum (FCS). The cell suspension was seeded into two 24-wells culture plates so that each well might contain l ml of the suspension and the culture plates were incubated in a $CO_2$-incubator.

After 24 hours, to each well was added l ml of HAT medium (RPMI l640 medium incorporated with 20% FCS containing l $\times$ l0$^{-4}$ M hypoxanthine, 4 $\times$ l0$^{-7}$ M aminopterin and l.6 $\times$ l0$^{-5}$ M thymidine). On the second day, third day, and thereafter every two days, one-half of the culture medium in each well was replaced by an equal volume of HAT medium. On the tenth day, one-half of the culture medium in each well was replaced by an equal volume of HT medium (HAT medium minus aminopterin). From the next day on, every two days, one-half of the culture medium in each well was replaced by an equal volume of ordinary medium (RPMI l640 medium plus l0% FCS). The culture supernatant collected on the l8th day was examined for anti-human C$_3$ antibody production.
3. Selection of hybridoma

For the purpose of selecting hybridoma capable of producing anti-human C3 antibody, each of the culture supernatants in the 48 wells was assayed by ELISA. First, each well of a 96-wells culture plate for ELISA was fed with 0.l ml of human C3 of l0 $\mu$g/ml in concentration and allowed to stand at 4°C for l6 hours to fix the human C3 to the culture plate. Each well was washed three times with a washing solution [l0 mM phosphate buffer, pH 7.4, containing 0.05% of Tween 20 (a nonionic surface active agent of Atlas Co.)]. Then, each well was fed with 0.2 ml of l% bovine serum albumin solution in order to avoid nonspecific absorption of the proteins in the culture supernatant, and was allowed to stand at 37°C for 2 hours. After washing three times with the washing solution, each well was fed with 0.l ml of the cell culture supernatant and allowed to stand at 37°C for 2 hours. For a negative reference, 0.l ml of RPMI l640 medium containing 20% FCS was fed. Individual wells were washed three times with the washing solution, then fed with 0.l ml of a solution of anti-mouse-immunoglobulin antibody labeled with peroxidase, and allowed to stand at 37°C for 2 hours. After washing three times with the washing solution, 0.l ml of a 0.4% o-phenylenediamine solution was fed and the reaction was carried out for 2 minutes at room temperature. The reaction was stopped by adding 0.05 ml of 6N sulfuric acid and absorbance at 490 nm was measured. The hybridoma growing in a culture supernatant which showed an absorbance twice or more as large as that of the negative reference was selected as the hybridoma capable of producing an anti-human C3 antibody. Among the 48 wells tested, six wells showed the production of anti-human C3 antibody.

4. Cloning

In order to prepare feeder cells, the thymus was removed from a BALB/c mouse (female, 6 weeks old) and was minced in a plastic Petri dish containing 10 ml of RPMI 1640 medium to obtain thymocytes. The thymocytes were washed three times with RPMI 1640 medium by repeated centrifugation (1,000 rpm for 10 minutes) and resuspended in 50 ml of RPMI 1640 medium containing 20% FCS. To the resulting cell suspension was added 0.1 ml of a hybridoma solution containing 500 cells/ml capable of producing anti-human C3 antibodies. After thorough mixing, the resulting mixture was seeded into a 96-wells culture plate so that each well might contain 0.2 ml of the mixture, and the culture plate was incubated for 10 days in a $CO_2$-incubator. The culture supernatants, in which the growth of cells was noticed, were assayed by ELISA to sort out 12 clones of hybridoma capable of producing anti-human C3 antibodies. Among these clones, one clone was again subjected to the second cloning in the same manner as described above to yield 5 clones of hybridoma capable of producing anti-human C3 antibodies, whereby complete cloning was achieved. ·

5. Preparation of monoclonal antibodies

In 0.2 ml of RPMI 1640 medium were suspended $2 \times 10^5$ cells of the clone C3-1 obtained by the steps from 1 to 4 described above, and the resulting suspension was intraperitoneally administered to BALB/c mouse (male, 6 weeks of age), after which the ascites was collected. To 5 ml of the ascites was added gradually a saturated ammonium sulfate solution until the final ammonium sulfate concentration had reached 50%. After the resulting mixture was stirred at room temperature for 2 hours, the precipitate was collected by centrifugation (3,000 rpm, 10 minutes) and then dissolved in 5 ml of physiological saline. Serial 10-fold dilutions of the resulting solution were assayed for the antibody activity by ELISA. The maximum dilution at which the antibody activity was detectable was $10^6$. The mouse IgG content (a measure of the antibody titer) was found to be 26 mg/ml as measured by use of an agarose plate containing rabbit anti-mouse IgG antibody (SRID method).

6. The clone C3-7 obtained by the steps from 1 to 4 described above was subjected to the same procedure as in the above step 5 to obtain 5 ml of a monoclonal antibody. The antibody activity and the mouse IgG content were measured in the same manner as in the above step 5 to find that the antibody activity was $10^5$ and the mouse IgG content 16 mg/ml.

7. The clone C3-9 obtained by the steps from 1 to 4 described above was subjected to the same procedure as in the above step 5 to obtain 5 ml of a monoclonal antibody. The antibody activity and the mouse IgG content were measured in the same manner as in the above step 5 to find that the antibody activity was $10^6$ and the mouse IgG content 23 mg/ml.

Referential Example 2

Preparation of monoclonal anti-human C4 antibody and hybridoma producing the same.

An immunogen was prepared by mixing a solution of 100 μg of human C4 in 0.1 ml of a 0.15 M sodium chloride solution with 0.1 ml of complete Freund's adjuvant. To a BALB/c mouse (male, 5 weeks old) was intraperitoneally administered 0.2 ml of the emulsion thus obtained. After 4 weeks, a solution of 100 μg of human C4 in 0.2 ml of a 0.15 M sodium chloride solution was injected into the tail vein of the mouse. Thereafter, in a manner similar to that described in the steps 2 to 4 of Referential Example 1, 5 clones of hybridoma capable of producing anti-human C4 antibodies were obtained. In a manner similar to the described in the step 5 of Referential Example 1, 6 monoclonal anti-human C4 antibodies (C4-1, C4-2, C4-5, C4-8, C4-17 and C4-32) were obtained. The antibody activities and the mouse IgG contents (a measure of the antibody titers) of the individual clones were as shown in Table 1.

## Table 1

| Anti-human C4 clone No. | Antibody activity | Mouse IgG content |
|---|---|---|
| C4-1 | $10^6$ | 19 (mg/ml) |
| C4-2 | $10^5$ | 23 |
| C4-5 | $10^5$ | 20 |
| C4-8 | $10^6$ | 24 |
| C4-17 | $10^5$ | 16 |
| C4-32 | $10^6$ | 18 |

Example I

Estimation of human serum C3 using monoclonal antibodies.
Reagents: The following reagents were prepared.

1. Buffer:

| | |
|---|---|
| Polyethylene glycol 6,000 | 4 g |
| Sodium chloride | 0.9 g |
| 0.01 M phosphate buffer (pH 7.0) | 100 ml |

2. Antibody solution:

| | |
|---|---|
| Monoclonal antibody C3-1 obtained in Referential Example 1 | 0.3 ml |
| Monoclonal antibody C3-7 obtained in Referential Example 1 | 0.3 ml |
| Buffer | 100 ml |

3. Standard serum:

Standard serum having a C3 content of 150 mg/dl.
Samples: Human sera
Procedures: In a test tube was placed 20 $\mu$l of the undiluted standard serum or each undiluted human serum, and 3 ml of the antibody solution was added, after which the resulting mixture was subjected to reaction at 37°C for 10 minutes, and then absorbance at 505 nm was measured by means of a spectrophotometer (HITACHI 624, a trade name, mfd. by Hitachi, Ltd.). Separately, 3 ml of the buffer solution containing no antibody was added to 20 µl of the undiluted standard serum or each undiluted human serum, and each of the mixtures thus obtained was incubated at 37°C for 10 minutes, after which absorbance at 505 nm was measured by means of the spectrophotometer, the path length in the cuvette being 10 mm.

Calculation:

$$\text{C3 content of human serum (mg/dl)} = \frac{E_S - (E_B + E_{S \cdot B})}{E_{Std} - (E_B + E_{Std \cdot B})} \times 150$$

wherein

$E_S$ : Absorbance of a reaction mixture of human serum and antibody solution.

$E_{S \bullet B}$ : Absorbance of a mixture of human serum and buffer.

$E_{Std}$ : Absorbance of a reaction mixture of standard serum and antibody solution.

$E_{Std \bullet B}$ : Absorbance of a mixture of standard serum and buffer.

$E_B$ : Absorbance of antibody solution.

In Table 2, there is made a comparison between the C3 contents of the human serum samples used in this Example calculated by the above equation and those determined by the SRID method.

Table 2

| Assay method / Human serum No. | Method of this invention, y | Method of SRID, x |
|---|---|---|
| 1 | 84 (mg/dl) | 82 (mg/dl) |
| 2 | 131 | 130 |
| 3 | 94 | 95 |
| 4 | 80 | 82 |
| 5 | 107 | 110 |
| 6 | 125 | 124 |
| 7 | 60 | 58 |
| 8 | 107 | 110 |
| 9 | 72 | 70 |
| 10 | 88 | 90 |

$$\gamma = 0.996$$

$$y = 0.971x + 2.4$$

It can be seen from Table 2 that there is a good correlation between the results obtained by the present method and those obtained by the SRID method.

Example 2

Estimation of human serum C4 using monoclonal antibodies.
Reagents: The following reagents were prepared.

### 1. Buffer;

| | |
|---|---|
| Polyethylene glycol 6,000 | 4 g |
| Sodium chloride | 0.9 g |
| 0.01 M veronal buffer (pH 7.5) | 100 ml |

### 2. Antibody solution;

| | |
|---|---|
| Monoclonal antibody C4-2 obtained in Referential Example 2 | 0.1 ml |
| Monoclonal antibody C4-17 obtained in Referential Example 2 | 0.1 ml |
| Monoclonal antibody C4-32 obtained in Referential Example 2 | 0.1 ml |
| Buffer | 100 ml |

Samples: Human sera having C4 contents of 10, 20, 40 and 60 mg/dl, respectively, were used.

Procedures: In a test tube was placed 20 $\mu$l of each undiluted human serum, and 2 ml of the antibody solution was added, after which the resulting mixture was subjected to reaction at 37°C for 10 minutes, and then absorbance at a wavelength of 340 nm was measured by means of a spectrophotometer (Type UV-200 of Shimadzu Seisakusho, Ltd.), the path length in the cuvette being 10 mm. Separately, 20 $\mu$l of each undiluted human serum was placed in a test tube and 2 ml of the buffer solution was added, after which the resulting mixture was incubated at 37°C for 10 minutes, and then absorbance at 340 nm was measured by means of the spectrophotometer.

Calculation: Absorbance ($E_S$) after the serum C4 and the antibody had reacted was calculated by the following equation;

$$E_S = E_{S \bullet A} - E_{S \bullet B}$$

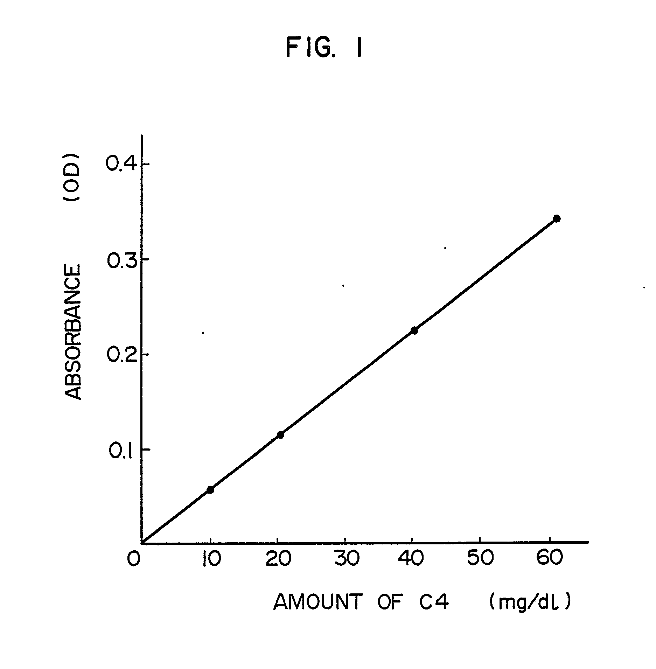

wherein $E_{S \bullet A}$: Absorbance of a reaction mixture of human serum and antibody solution.
$E_{S \bullet B}$: Absorbance of a mixture of human serum and buffer.
The measurement results are shown in Fig. 1.

Example 3

Estimation of human serum C4 using monoclonal antibodies.
Reagents: The following reagents were prepared.

1.  Buffer:

|  |  |
|---|---|
| Polyethylene glycol 6,000 | 4 g |
| Sodium chloride | 0.8 g |
| 0.01 M Tris-HCl buffer (pH 7.5) | 100 ml |

2.  Antibody solution:

|  |  |
|---|---|
| Monoclonal antibody C4-5 obtained in Referential Example 2 | 0.1 ml |
| Monoclonal antibody C4-32 obtained in Referential Example 2 | 0.1 ml |
| Buffer | 50 ml |

3. Standard sera:

Standard sera having C4 contents of l0, 20, 40, 60 and 80 mg/dl, respectively.
Samples: Human sera

Procedures: In a cuvette was placed l0 $\mu$l of each undiluted standard serum or each undiluted human serum, and 500 $\mu$l of the antibody solution was added, after which the resulting mixture was subjected to reaction of room temperature for l5 minutes, and then the scattered light intensity at a wavelength of 633 nm was measured by means of a laser nephilometer (ZD-80l). Separately, the same measurement as described above was carried out, except that the buffer solution was used in place of the antibody solution. The intensity of scattered light ($D_S$) after the human C4 and the antibody had reacted was calculated by the following equation:

$$D_S = D_{S \bullet A} - D_{S \bullet B}$$

wherein $D_{S \bullet A}$ : Scattered light intensity (mV) of a reaction mixture of serum and antibody solution.
$D_{S \bullet B}$ : Scattered light intensity (mV) of a mixture of serum and buffer.
A calibration curve obtained by using the standard sera is shown in Fig. 2. In Table 3, there is made a comparison between the C4 contents of the human serum samples used in this Example determined from Fig. 2 and those determined by the SRID method.

Table 3

| Assay method<br>Human serum No. | Method of this invention, y | Method of SRID, x |
|---|---|---|
| 1 | 26 (mg/dl) | 26 (mg/dl) |
| 2 | 29 | 28 |
| 3 | 28 | 28 |
| 4 | 30 | 30 |
| 5 | 22 | 22 |
| 6 | 25 | 24 |
| 7 | 16 | 16 |
| 8 | 34 | 34 |
| 9 | 38 | 38 |
| 10 | 26 | 24 |
| 11 | 18 | 18 |
| 12 | 24 | 24 |

$$\gamma = 0.994$$

$$y = 0.991x + 0.5$$

It can be seen from Table 3 that there is a good correlation between the results obtained by the present method and those obtained by the method of SRID.

**Claims**

1. A method for the determination of complements in a living body sample comprising exposing an antigen-antibody complex to light, and measuring the change of optical properties, characterized by using the living body sample without dilution, and using two or more kinds of monoclonal antibodies to human complements as antibodies to the complement being assayed.

2. A method for the determination according to Claim I, wherein the monoclonal antibodies are monoclonal antibodies to human complements produced by a hybridoma formed by the fusion between cells derived from a mouse myeloma line and spleen cells of mouse immunized with human complement being assayed.

3. A method for the determination according to Claim I, wherein the change of optical properties is that of light-scattering intensity.

4. A method for the determination according to Claim I, wherein the change of optical properties is that in transmittance.

5. A method for the determination according to Claim I, wherein the complement is human C3 or human C4.

FIG. 1

FIG. 2